Europäisches Patentamt

European Patent Office

Office européen des brevets

(11) Veröffentlichungsnummer: **0 433 607 A1**

# (12) EUROPÄISCHE PATENTANMELDUNG

(21) Anmeldenummer: 90120378.6

(22) Anmeldetag: 24.10.90

(51) Int. Cl.5: **A61B 17/22**

(30) Priorität: 18.12.89 DE 3941683

(43) Veröffentlichungstag der Anmeldung:
26.06.91 Patentblatt 91/26

(84) Benannte Vertragsstaaten:
CH ES FR IT LI NL

(71) Anmelder: **DORNIER MEDIZINTECHNIK GMBH**
**Postfach 1128**
**W-8000 München(DE)**

(72) Erfinder: **Viebach, Thomas, Dipl.-Ing.**
**Kapellanger 8**
**W-8081 Pischertshofen(DE)**
Erfinder: **Kreibich, Rainer**
**Ysenburgstrasse 12**
**W-8000 München 19(DE)**
Erfinder: **Nuber, Bernd, Dipl.-Ing.**
**Karlstrasse 120**
**W-8000 München(DE)**
Erfinder: **Pauker, Robert, Dipl.-Ing.**
**Westendstrasse 12**
**W-8316 Frontenhausen(DE)**
Erfinder: **Hesse, Alexander, Dr.-Ing.**
**Güntherstrasse 15**
**W-8000 München 19(DE)**

(74) Vertreter: **Landsmann, Ralf, Dipl.-Ing.**
**c/o DORNIER GMBH Postfach 1420**
**W-7990 Friedrichshafen 1(DE)**

(54) Ultraschallortung für die Lithotripsie.

(57) Für die Lithotripsie mit extrakorporal erzeugten Stosswellen wird eine Haltevorrichtung (H) für den zum Orten benutzten Ultraschall-Transducer ($T_1$) vorgeschlagen. Damit ist die Lage des Ultraschall-Transducers ($T_1$) relativ zum Therapiekopf (TK) und relativ zur Patientenliege (L) festlegbar. (Figur)

EP 0 433 607 A1

## ULTRASCHALLORTUNG FÜR DIE LITHOTRIPSIE

Die Erfindung betrifft eine Vorrichtung zum Behandeln von Patienten mit extrakorporal erzeugten Stosswellen, insbesondere für die betührungsfreie Lithotripsie nach dem Oberbegriff des Anspruchs 1.

Eine solche Vorrichtung ist bereits in der DE-Patentanmeldung 39 15 382.7 vorgeschlagen. Bei diesem Lithotripter ist der Ortungsarm am Therapiekopf angeordnet. Es ist ein Bezug der mit dem Ortungsarm erhaltenen Meßdaten zum Therapiefokus direkt gegeben. Die Anforderungen an die Mechanik des Gerätes sind wesentlich geringer als bei Ortungsarmen, die keine feste Verbindung zum Therapiekopf haben.

Dieser Vorteil wird jedoch durch die Problematik der Relativbewegungen des arretierten Armes zum Patienten vermindert. Neben dem Problem, daß ein einmal eingestellter Stein aus dem Ultraschall-Bild verlorengeht, ist noch auf eine Patientengefährdung durch Kollisionen zu achten.

Eine andere Möglichkeit ist die Anordung des Ortungsarmes an der Patientenliege (DE-Gm 88 00 985). Der Vorteil dieser Anbindung liegt darin, daß bei gefundenem Stein der Arm an der Patientenoberfläche arretiert werden kann, ohne daß sich bei der Positionierung der Patientenliege relativ zum Therapiekopf die Lage des Armes zum Patienten verändert. Der Schallkopf bleibt damit angekoppelt, der Bildausschnitt ändert sich nicht, und es besteht keine Kollisionsgefahr. Nachteilig wirkt sich bei dieser Anordnung die Notwendigkeit zur Lagebestimmung des Therapiefokus relativ zur Liege aus. Neben der Ungenauigkeit bei der Erfassung dieser Größen geht eine eventuelle Verformung bei Belastung der Liege direkt in den Meßfehler des Ortungsarmes ein.

Ein an der Liege angebrachter Ortungsarm ist in bezug auf die Ortungsgenauigkeit stets einem an der Therapieeinheit angebrachten unterlegen, während ein an der Therapieeinheit angebrachter Arm die Kontrolle während der Positionierbewegungen nur schlechter ermöglicht. Weiterhin ist die Realisierung eines hochgenauen und gleichzeitig bremsbaren Ortungsarmes oft nur in Form einer verhältnismäßig komplexen und damit in der Regel schweren und unflexiblen Mechanik möglich.

Aus der DE-C-34 27 001 ist ein Lithotripter bekannt, bei dem ein Ortungsgerät am Patienten oder seiner Liege befestigt ist und ein anderes Ortungsgerät am Therapiekopf angeordnet ist. Diese Vorrichtung ist jedoch relativ unhandlich. Auch ist die Bewegungsmöglichkeit des zweiten Ortungsgerätes sehr stark eingeschränkt.

Aufgabe der Erfindung ist es, eine Vorrichtung zum Behandlen von Patienten mit extrakorporal erzeugten Stosswellen vorzuschlagen, bei der die Ortung des zu behandelnden Körperteils exakt in bezug auf den Behandlungsfokus der Therapieeinheit erfolgt und zur Behandlungskontrolle die Lage des Schallkopfes bezüglich des Patienten festliegt.

Diese Aufgabe wird erfindungsgemäß gelöst von einer Vorrichtung mit den Merkmalen des Anspruchs 1. Ausgestaltungen der Erfindung sind Gegenstände von Unteransprüchen.

Kern der Erfindung ist es, die beiden ursprünglich schwer zu vereinbarenden Anforderungen auch physikalisch voneinander zu trennen. Durch die erfindungsgemäße gebremste oder bremsbare Haltevorrichtung kann der Ultraschall-Transducer nun auch gegenüber der Patientenliege fixiert werden. Unter einer gebremsten oder bremsbaren Haltevorrichtung ist dabei ein Arm zu verstehen, der Vorrichtungen zum Bremsen von Bewegungen der Einzelteile des Arms untereinander aufweist.

Durch die Erfindung werden die beiden widersprechenden Teilaufgaben gelöst:

1. Für die Ortung wird erreicht, daß die Ortungsvorrichtung die Lage des zu behandelnden Organs (z.B. des Steines) relativ zum Behandlungsfokus der Therapieeinheit exakt erfasst. Das zur Verwendung kommende Wegaufnehmersystem muß dazu die Lage des Ultraschall-Transducers relativ zur Therapieeinheit bestimmen, was bei Verwendung eines Gelenkarmes zweckmäßig dadurch erreichbar ist, daß der Gelenkarm direkt an der Therapieeinheit befestigt ist. Da bei einer Anbindung an die Patientenliege ein zusätzliches Wegaufnehmersystem vorhanden sein müsste, um die Relativposition zwischen Patientenliege und Therapieeinheit zu erfas sen, würde diese Verlängerung der Messkette durch die auftretenden Messfehler sowie durch eventuelle mechanische Unsicherheiten zu einer Verschlechterung der Ortungsgenauigkeit führen. Weiterhin ist die Ortungsvorrichtung in allen Freiheitsgraden leicht beweglich und schränkt den Arzt in der Bewegungsmöglichkeit des Ultraschall-Transducers nicht ein. Unter diesen Gesichtspunkten ist ein kleiner, leichtgewichtiger und sehr leicht beweglicher Gelenkarm vorteilhaft.

2. Für die Behandlungskontrolle wird erreicht, daß der Ultraschall-Transducer am Patientenkörper arretierbar ist. Damit kann das zu behandelnde Organ während der Dauer der Behandlung im Ultraschall-Bild dargestellt werden. Der Gelenkarm, der den Ultraschall-Transducer trägt, ist dabei liegenfest gehaltert, um bei Positionierbewegungen nicht relativ zum Patienten verschoben zu werden.

In einer vorteilhaften Ausführung ist der Ortungsarm leichtgewichtig und sehr beweglich gebaut.

Eine Ausführungsform der Erfindung wird anhand einer Figur näher erläutert.

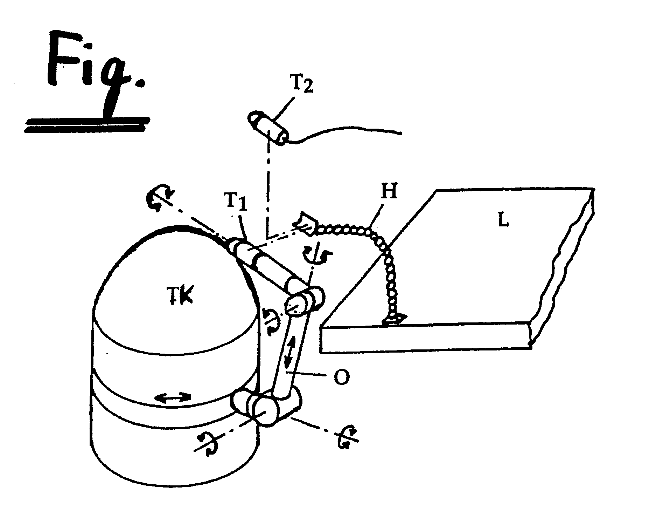

Die Figur zeigt den Therapiekopf TK, an dem am Ortungsarm O ein Transducer $T_1$ befestigt ist. Die Bewegungsmöglichkeiten des Ortungsarmes O sind durch Pfeile angedeutet. So kann der Ortungsarm O eine Kreisbewegung um die Längsachse des Therapiekopfes vornehmen, Schwenkbewegungen in den beiden gezeigten Armgelenken durchführen. Eine weitere Bewegungsmöglichkeit ist die Verlängerung des ersten Armelementes. Zusätzlich ist eine Drehbarkeit des zweiten Armgelenkes bezüglich des ersten Armgelenkes gegeben, eine Drehbeweglichkeit beider Gelenke bezüglich einer waagrecht verlaufenden Achse und eine Drehmöglichkeit des Transducers $T_1$ um seine eigene Achse. Der Schallkopf $T_1$ besitzt damit alle Freiheitsgrade der Bewegung im Behandlungsbereich. Weiter gezeigt ist die Patientenliege L mit der erfindungsgemäßen Haltevorrichtung H, an der der Transducer $T_1$ oder ein weiterer freier Transducer $T_2$ befestigbar ist.

Erfindungsgemäß ist der Ortungsarm O am Therapiekopf TK so angebracht, daß über diesen Ortungsarm die Lage des Ultraschall-Transducers $T_1$ und damit des zu behandelnden Organs oder des Steins erfasst werden kann. Dieser Arm O, mit den integrierten Weg- und Winkelaufnehmern, dient hier ausschließlich zur Koordinatenbestimmung bei der Ortung und ist nicht bremsbar ausgeführt. Dafür ist bei seiner Konstruktion auf geringes Gewicht und größtmögliche Beweglichkeit geachtet worden. Die Aufgabe der Schallkopfarretierung übernimmt die erfindungsgemäße Haltevorrichtung H an der Liege L. An dieser Halterung kann bei Bedarf der ungebremste Ortungsarm O angebracht werden, um ihn gegenüber dem Patienten zu fixieren. Ebenso ist es möglich, an der Haltevorrichtung H einen freien Schallkopf $T_2$ - also einen ohne Ortungsarm -, zur Behandlungskontrolle am Patienten zu fixieren.

## Ansprüche

1. Vorrichtung zur Behandlung von Patienten mit extrakorporal erzeugten Stosswellen, insbesondere für die berührungsfreie Lithotripsie, mit
   - einer Patientenliege (L)
   - einem Therapiekopf (TK) zur Erzeugung und Einleitung von Stosswellen in den Patientenkörper
   - einem Ultraschall-Transducer ($T_1$), der an einem Ortungsarm (O) beweglich gegenüber dem oder am Therapiekopf (TK) befestigt ist und
   dessen Position über Messwertaufnehmer und/oder über akustische oder optische Positionsbestimmung relativ zum Therapiekopf (TK) bestimmbar ist, **gekennzeichnet durch** eine gebremste oder bremsbare Haltevorrichtung (H) für den Transducer ($T_1$) an der Patientenliege (L).

2. Vorrichtung nach Anspruch 1, **dadurch gekennzeichnet**, daß der Ortungsarm (O) leicht und leicht beweglich ist.

3. Vorrichtung nach einem der vorhergehenden Ansprüche, **dadurch gekennzeichnet**, daß der Ortungsarm (O) ungebremst ist.

4. Vorrichtung nach einem der vorhergehenden Ansprüche, **dadurch gekennzeichnet**, daß der Ortungsarm (O) alle Freiheitsgrade der Bewegung im Raum besitzt.

5. Vorrichtung nach einem der vorhergehenden Ansprüche, **dadurch gekennzeichnet**, daß die Haltevorrichtung (H) als in allen Lagen arretierbares Stativ, als bremsbarer Gliederarm oder in allen Richtungen biegsamer sogenannter "Schwanenhals" ausgebildet ist.

6. Vorrichtung nach einem der vorhergehenden Ansprüche, **dadurch gekennzeichnet**, daß die Haltevorrichtung (H) lösbar mit dem Schallkopf ($T_1$) am Ortungsarm (O) verbunden ist und die Befestigung eines mechanisch ganz freien Schallkopfes ($T_2$) ermöglicht, so daß Ortungsarm (O) und Schallkopfhaltevorrichtung (H) auch unabhängig voneinander benützt werden können.

Fig.

# EUROPÄISCHER RECHERCHENBERICHT

Nummer der Anmeldung

## EP 90 12 0378

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A,D | EP-A-0 169 311 (DORNIER)<br>* Ansprüche 1,11; Figur 2 * | 1 | A 61 B<br>17/22 |
| A,D | DE-U-8 800 985 (DORNIER)<br>* Seite 3, Zeilen 6-17; Figur 1 * | 1 | |

| RECHERCHIERTE SACHGEBIETE (Int. Cl.5) |
|---|
| A 61 B |

Der vorliegende Recherchenbericht wurde für alle Patentansprüche erstellt

| Recherchenort | Abschlußdatum der Recherche | Prüfer |
|---|---|---|
| Den Haag | 21 März 91 | MOERS R.J. |